# EUROPEAN PATENT SPECIFICATION

(11) **EP 0 681 577 B1**
(45) Date of publication and mention of the grant of the patent: **15.09.1999**
(21) Application number: 95902843.2
(22) Date of filing: 25.11.1994
(51) Int. Cl.: C07D 311/56

(54) **PROCESS FOR THE PREPARATION OF SUBSTITUTED 4-HYDROXYCOUMARINS**
VERFAHREN ZUR HERSTELLUNG VON SUBSTITUIERTEN 4-HYDROXY-KUMARINE
PROCEDE DE PREPARATION DE 4-HYDROXYCOUMARINES SUBSTITUEES

(30) Priority: 30.11.1993 GB 9324517
(43) Date of publication of application: 15.11.1995
(73) Proprietor: GREAT LAKES (UK) LIMITED, Widnes, Cheshire WA8 8NS (GB)
(72) Inventor: TIMMS, Allan, William, South Wirral L65 6TR (GB); GANDY, Robert, Grassendale Liverpool L19 0ND (GB); WILSON, Stephen, Widnes Cheshire WA8 9AQ (GB)
(74) Representative: Lyons, Andrew John
(86) International application number: GB9402591
(87) International publication number: WO9515322

(56) References cited:
- GB-A- 1 458 670

## Description

This invention concerns a process for preparing substituted 4-hydroxycoumarins.

4-Hydroxycoumarins substituted in the 3-position having the following general formula (I) 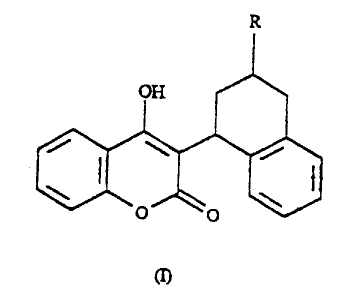 wherein, R is selected from hydrogen, phenyl, substituted phenyl, alkyl and substituted alkyl groups may be useful for controlling vermin and, in particular, rodents, since they can exhibit anticoagulant properties. As examples of compounds referred to in general formula (I), the following compounds are commercially available and extensively used in rodenticidal compositions:

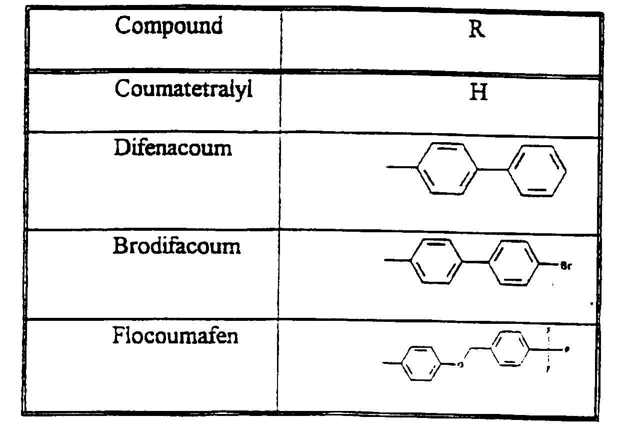

The prior art teaches that these compounds may be manufactured by condensation of 4-hydroxycoumarin with an appropriately substituted tetralin. For example, GB 1,458,670 demonstrates that compounds of general formula (I) may be prepared by condensation of 4-hydroxycoumarin with compounds of the general formula (II) (X = OH) without a solvent or in a solvent such as acetic acid in the presence of a dehydrating agent such as sulphuric acid or by condensation of 4-hydroxycoumarin with a compound of general formula (II) (X = halogen) with or without the use of a solvent. 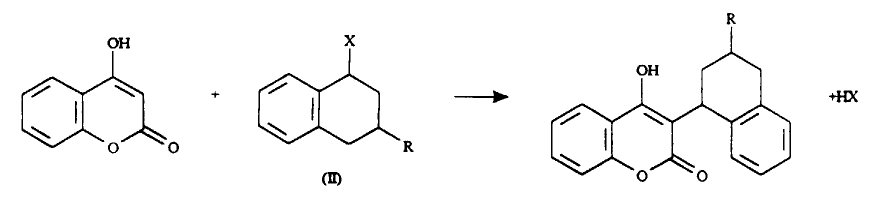 X = OH, halogen; R as defined above
GB 1,518,858 has further demonstrated the preparation of compounds of general formula (I) by the reaction of 4-hydroxycoumarin with compounds of general formula (III) in the presence of a suitable acidic catalyst. 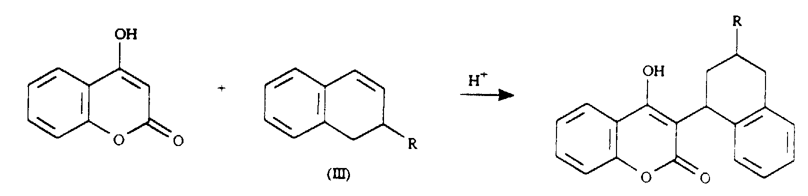 R as defined above
EP 177,080 has further modified the process for the manufacture of compounds of general formula (I) by reacting 4-hydroxycoumarin with compounds of general formula (II) in the presence of a catalyst and an organic solvent selected from formic acid, a mixture of formic acid and a least one C₂₋₆ aliphatic acid having a boiling temperature at atmospheric pressure in the range of 60°C to 105°C and a liquid chlorinated alkane having a boiling temperature in the range 60°C to 125°C and effecting the reaction at a temperature in the range of 60°C to the reflux temperature of the reaction mixture. The catalyst is preferably a sulphonic acid, especially selected from methanesulphonic acid, benzenesulphonic acid and p-toluenesulphonic acid.

Whereas any of the above methods may be used on an industrial scale to manufacture compounds of general formula (I), they all have scope for improvement. Although the process of EP 177080 can be utilised conveniently on manufacturing scale, it still utilises 1,2-dichloroethane, which is an environmentally harmful solvent.

An object of this invention is to provide an improved process for preparing substituted 4-hydroxycoumarins of the general formula (I) defined above.

We have surprisingly found that the reaction may be conducted in liquid aromatic hydrocarbons, such as toluene, xylene or benzene with similar or even better yields than hitherto and with a considerably easier work-up technique.

Accordingly this invention provides a process for preparing compounds of general formula (I) above wherein R is selected from hydrogen, phenyl, substituted phenyl, alkyl and substituted alkyl groups by reacting 4-hydroxycoumarin and a substituted tetralol of the general formula 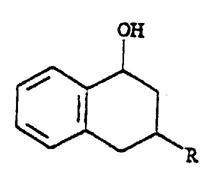 wherein R as defined above, in the presence of an organic solvent and a catalyst, characterised in that the solvent is an aromatic hydrocarbon.

The aromatic hydrocarbon solvent is preferably selected from benzene, toluene and xylene. The reaction is preferably carried out at the boiling point of the solvent and preferably with the azeotropic removal of water.

The catalyst for use in the process of the invention is preferably an acid, especially a sulphonic acid. Preferred sulphonic acid catalysts are p-toluenesulphonic acid, benzenesulphonic acid and methanesulphonic acid.

Yet another alternative preferred catalyst for use in the invention is a carboxylic acid, especially a strong carboxylic acid, such as trichloroacetic acid.

At the end of the reaction period, excess solvent may be removed by distillation and replaced by, for example methanol. After stirring in the now mixed solvent of methanol and aromatic hydrocarbon, such as toluene, benzene or xylene, for a few hours, the product crystallises and may be isolated by techniques such as vacuum filtration and washing. The use of such solvent systems described herein may considerably and significantly lessen the environmental impact of any vapours which may escape via the scrubbing system to atmosphere. Furthermore, when the process of this invention is utilised, no aqueous effluents need be produced, thereby preventing accidental discharge of water contaminated with trace amounts of organic materials to the sewerage system. For these reasons alone the process of the present invention represents a significant improvement over prior art processes.

A further improvement which may result from the process of the invention is that better yields of product may be realisable using smaller quantities of the relatively expensive raw material 4-hydroxycoumarin and the catalyst, such as, p-toluenesulphonic acid. Thus, in the method cited in the literature, 2.0 mols of 4-hydroxycoumarin / mol tetralol have to be utilised for a good reaction whereas in the method of the present invention the ratio may be less than 2:1, typically of the order of 1.5:1. The amount of catalyst, such as p-toluenesulphonic acid, may be reduced from 1.05 mols/mol tetralol to as low as 0.16 mols/mol. Whereas this reduction in amount of catalyst is not significant in financial terms, the modification represents a significant advance in the ease of work-up of the product.

The catalyst which is employed to bring about a good reaction is almost any strong acid. Previously, aromatic sulphonic acids have been successfully employed. These catalysts are adequate for the purpose but have the disadvantage of only having a small solubility in the solvent systems described herein. This may be the reason that much reduced quantities of, for example, p-toluenesulphonic acid are required in the present invention; the excess acid being insoluble in the reaction medium can take no part in the reaction. We have discovered that the more soluble trichloroacetic acid can act as the catalyst and give good reaction. This may ease the work-up conditions as the catalyst is removed in the solvent on filtration of the final product. It is thus anticipated that any strong acid of pKₐ of <=0.9 may be utilised in the reaction. The limiting factor in the choice of acid (apart from pKₐ value being <=0.9) is that it may either be quantitatively removed from the reaction medium by filtration or may have sufficient solubility in the reaction medium that it is removable in the solvent stream on isolation of the product.

The invention will now be illustrated by means of the following examples.

### Example 1

### Preparation of 3-(3-biphenyl-4-yl-1,2,3,4-tetrahydro-1-naphthyl)-4-hydroxycoumarin. (Difenacoum)

A mixture of toluene (100cm³) and p-toluenesulphonic acid (2.0g; 11.16 mmol) was heated together under reflux in a reaction vessel equipped with a Dean and Stark apparatus. Any water present was removed via the Dean and Stark apparatus. After cooling to <30°C 4-hydroxycoumarin (12.0g; 74 mmol) and 3-(biphenyl-4-yl)-1,2,3,4-tetrahydro-1-naphthol (15.0g; 50 mmol) were added. The mixture was heated to reflux again and water removed via the Dean and Stark apparatus. Heating was continued for 20 hours when the reaction was deemed to be complete. Toluene (75cm³) was removed by distillation and methanol (100cm³) added. Boiling under reflux was continued for a further 2 hours during which time crystallisation occurred. The suspension was cooled to <20°C and aged at this temperature overnight. The suspension was filtered and washed with methanol (2x20cm³). Difenacoum (19.4g; 87.4%) of satisfactory quality was obtained.

### Example 2

### Preparation of 3-[3-(4'-bromobiphenyl-4-yl)-1,2,3,4-tetrahydro-1-naphthyl]-4-hyroxycoumarin. (Brodifacoum)

By the method described in Example 1, Brodifacoum was prepared in 94% yield based on 3-(4'-bromodiphenyl-4-yl)-1,2,3,4-tetrahydro-1-naphthol.

## Claims

1. A process for preparing compounds of general formula (I) 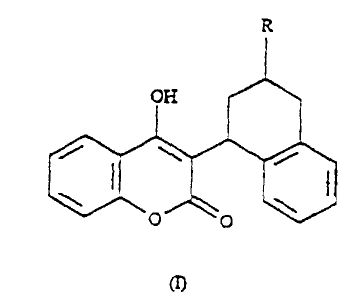 wherein R is selected from hydrogen, phenyl, substituted phenyl, alkyl groups and substituted alkyl groups by reacting 4-hydroxycoumarin and a substituted tetralol of the general formula 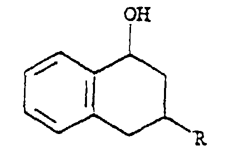 wherein R is as defined above, in the presence of an organic solvent and a catalyst, characterised in that the solvent is an aromatic hydrocarbon.

2. A process as claimed in claim 1, characterised in that the solvent is selected from benzene, toluene and xylene.

3. A process as claimed in claim 1 and 2, characterised in that the reaction is carried out at the boiling point of the solvent.

4. A process as claimed in claim 1, 2 or 3, characterised in that the reaction is carried out with the azeotropic removal of water.

5. A process as claimed in any one of the preceding claims, characterised in that the catalyst is a sulphonic acid.

6. A process as claimed in claim 5, characterised in that the catalyst is selected from p-toluenesulphonic acid, benzenesulphonic acid and methanesulphonic acid.

7. A process as claimed in any one of claims 1 to 4, characterised in that the catalyst is a strong carboxylic acid.

8. A process as claimed in claim 6, characterised in that the catalyst is trichloroacetic acid.

9. A process as claimed in any one of the preceding claims, characterised in that the reaction is worked up by addition of a precipitant.

10. A process as claimed in claim 9, characterised in that the precipitant is methanol.

11. A process as claimed in any one of the preceding claims, wherein R is hydrogen.

12. A process as claimed in any one of the preceding claims, wherein R is

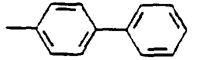

13. A process as claimed in any one of the preceding claims, wherein R is

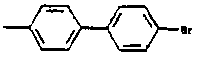

14. A process as claimed in any one of the preceding claims, wherein R is

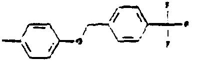

## Patentansprüche

1. Verfahren zur Herstellung von Verbindungen der allgemeinen Formel (I) 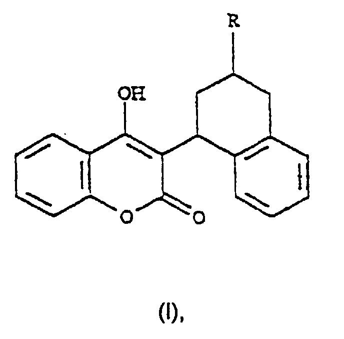 wobei R aus Wasserstoff, Phenyl, substituiertem Phenyl, Alkylgruppen und substituierten Alkylgruppen ausgewählt ist,
durch Reaktion von 4-Hydroxycumarin und einem substituiertem Tetralol der allgemeinen Formel 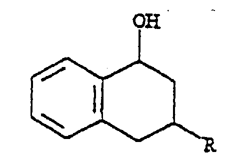 wobei R definiert ist wie oben,
in Gegenwart eines organischen Lösungsmittels und eines Katalysators, gekennzeichnet dadurch, daß
das Lösungsmittel ein aromatischer Kohlenwasserstoff ist.

2. Verfahren nach Anspruch 1, dadurch gekennzeichnet, daß
das Lösungsmittel aus Benzol, Toluol und Xylol ausgewählt ist.

3. Verfahren nach Anspruch 1 oder 2, dadurch gekennzeichnet, daß
die Reaktion beim Siedepunkt des Lösungsmittels ausgeführt wird.

4. Verfahren nach einem der Ansprüche 1, 2 oder 3, dadurch gekennzeichnet, daß
die Reaktion unter azeotroper Wasserentfernung durchgeführt wird.

5. Verfahren nach einem der vorangehenden Ansprüche, dadurch gekennzeichnet, daß
der Katalysator eine Sulfonsäure ist.

6. Verfahren nach Anspruch 5, dadurch gekennzeichnet, daß
der Katalysator aus p-Toluolsulfonsäure, Benzolsulfonsäure und Methansulfonsäure ausgewählt ist.

7. Verfahren nach einem der Ansprüche 1 bis 4, dadurch gekennzeichnet, daß
der Katalysator eine starke Carbonsäure ist.

8. Verfahren nach Anspruch 6, dadurch gekennzeichnet, daß
der Katalysator Trichloressigsäure ist.

9. Verfahren nach einem der vorangehenden Ansprüche, dadurch gekennzeichnet, daß
die Reaktion durch die Zugabe eines Fällungsmittels aufgearbeitet wird.

10. Verfahren nach Anspruch 9, dadurch gekennzeichnet, daß
das Fällungsmittel Methanol ist.

11. Verfahren nach einem der vorangehenden Ansprüche, wobei
R Wasserstoff ist.

12. Verfahren nach einem der vorangehenden Ansprüche, wobei
R 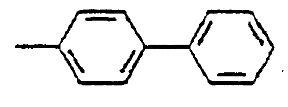 ist.

13. Verfahren nach einem der vorangehenden Ansprüche, wobei
R 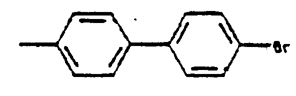 ist.

14. Verfahren nach einem der vorangehenden Ansprüche, wobei
R 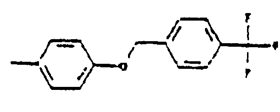 ist.

## Revendications

1. Un procédé de préparation de composé de formule générale (I) 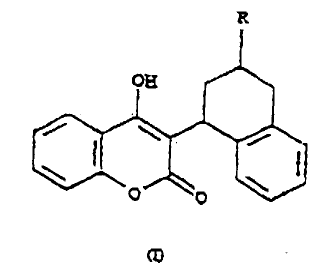 dans laquelle R est choisi parmi hydrogène, groupes phényle, phényle substitué, alkyle et groupe alkyle substitué, par réaction de la 4-hydroxycoumarine et d'un tétralol substitué de formule générale 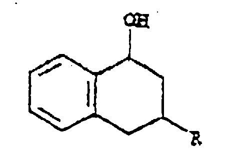 dans laquelle R tel que défini ci-dessus, en présence d'un solvant organique et d'un catalyseur, caractérisé en ce que le solvant est un hydrocarbure aromatique.

2. Un procédé selon la revendication 1, caractérisé en ce que le solvant choisi parmi benzène, toluène et xylène.

3. Un procédé selon l'une quelconque des revendications 1 et 2, caractérisé en ce qu'on effectue la réaction au point d'ébullition du solvant.

4. Un procédé selon la revendication 1, 2 ou 3, caractérisé en ce qu'on effectue la réaction avec élimination azéotropique d'eau.

5. Un procédé selon une quelconque des revendications précédentes, caractérisé en ce que le catalyseur est un acide sulfonique.

6. Un procédé selon la revendication 5, caractérisé en ce que le catalyseur est choisi parmi l'acide p-toluènesulfonique, l'acide benzènesulfonique et l'acide méthanesulfonique.

7. Un procédé selon une quelconque des revendications 1 à 4, caractérisé en ce que le catalyseur est un acide carboxylique fort.

8. Un procédé selon la revendication 6, caractérisé en ce que le catalyseur est l'acide trichloroacétique.

9. Un procédé selon une quelconque des revendications précédentes, caractérisé en ce qu'on traite la réaction par addition d'un agent précipitant.

10. Un procédé selon la revendication 9, caractérisé en ce que l'agent précipitant est le méthanol.

11. Un procédé selon une quelconque des revendications précédentes, dans lequel R est l'hydrogène.

12. Un procédé selon une quelconque des revendications précédentes, dans lequel R est

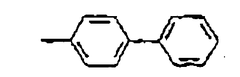

13. Un procédé selon une quelconque des revendications précédentes, dans lequel R est

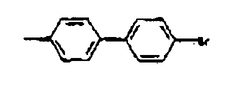

14. Un procédé selon une quelconque des revendications précédentes, dans lequel R est
